# EUROPEAN PATENT APPLICATION

(11) **EP 0 541 859 A1**
(43) Date of publication of application: **19.05.1993**
(21) Application number: 91310385.9
(22) Date of filing: 11.11.1991
(51) Int. Cl.: A61F 15/02

(54) **Electric plaster cast saw control system**

(71) Applicant: Yang, Chang-Min, Tainan (TW); Chou, Chun-Mei, Tainan (TW)
(72) Inventor: Yang, Chang-Min, Tainan (TW); Chou, Chun-Mei, Tainan (TW)
(74) Representative: W.P. THOMPSON & CO.

(57) **Abstract**

An electric plaster cast saw control system for cutting a plaster cast (80) composed of an inner layer (82) of absorbent cotton and an outer layer (84) of hardened covering material includes a thin conductive plate (10) placed between the inner layer and the outer layer, and a relay device triggered to cut off the power supply to the electric saw (86) on the instant that the saw blade makes contact with the thin conductive plate, thereby to stop the electric saw instantaneously. A reset device permits the power supply to be restored to the electric saw either automatically or under the control of the operator after cutting off the power source.

## Description

This invention relates to a control system for electric plaster cast saws, and particularly to a control system to stop electric plaster cast saws automatically and promptly during the cutting of a plaster cast or other covering material.

Human limbs may be fractured or may be deformed either congenitally or due to accident or illness. Currently, the medical treatment for dealing with bone fracture or deformity always involves wrapping of medical plaster around the injured area and allowing the plaster to harden so that the limb can heal quickly without external influences.

After a certain period, the plaster cast surrounding the injured limb must be cut with an electric saw for removal of the cast and examination of the limb. The limb may have to receive other therapy or surgery if necessary.

In general, the depth to which the saw has cut into the plaster cast is judged by visual inspection, the accuracy of which depends on the experience of the operator. Hence, skill is indispensable if one is to avoid injury to the patient. However, even if the sawing is carried out carefully, the patient's skin or flesh may be hurt accidentally. Such accidents occur frequently. Therefore, patients are always afraid of such sawing, the operator may hesitate to carry out the sawing, and consequently the operation is prolonged. Accidental damage to the patient may result from unexpected movement of the patient or uncontrolled shaking of the limb, especially when the patient is a baby or person who has lost the capacity to control his or her body.

Therefore, there is an urgent need in the medical field for a controller which can stop an electric saw automatically as soon as the plaster cast is cut through.

It is the main object of the present invention to provide a control system for an electric plaster cast saw to stop the electric saw automatically on the instant that the plaster cast is cut through in order to prevent injury to the patient.

Another object of the present invention is to provide a control system for an electric plaster cast saw which can effectively prevent injury to a patient's skin or flesh, so that the patient's fear can be eliminated, the operator's confidence for safe operation is improved, and unnecessary disputes can be avoided.

Another object of the present invention is to provide a control system for an electric plaster cast saw which can cut off the power supply and stop the electric saw automatically on the instant that the plaster cast is cut through, so that cutting off of the plaster cast can be performed by any nurse or operator and the burden on experienced nurses or surgeons can be relieved.

The electric plaster cast saw control system according to the present invention includes a thin conductive plate between the plaster layer and absorbent cotton layer of each plaster cast with an end of the thin conductive plate exposed outside the plaster cast so that it can be connected to control means by a wire. On the instant that the electric saw makes contact with the thin conductive plate, a relay is triggered to trip from its normal closed contact to normal open contact, and consequently to cut off the power supply to the electric saw instantaneously. The electric saw is thus stopped immediately to prevent injury to the patient. The control system preferably includes a reset device to resume power supply to the electric saw, and a warning device to give an audible or visual warning signal.

These and other objects and features of the invention will become more apparent from the following description taken in connection with the accompanying drawings, wherein:
Fig. 1 is a perspective view of a first embodiment of electric plaster cast saw control system according to the present invention;
Fig. 2 is a perspective view of a second embodiment of electric plaster cast saw control system according to the present invention, in which an automatic reset device replaces the manual reset device shown in Fig. 1;
Fig. 3 is a sectional view taken along line 3-3 in Fig. 1;
Fig. 4 is a block diagram for the first embodiment of the present invention; and,
Fig. 5 is a block diagram for the second embodiment of the present invention.

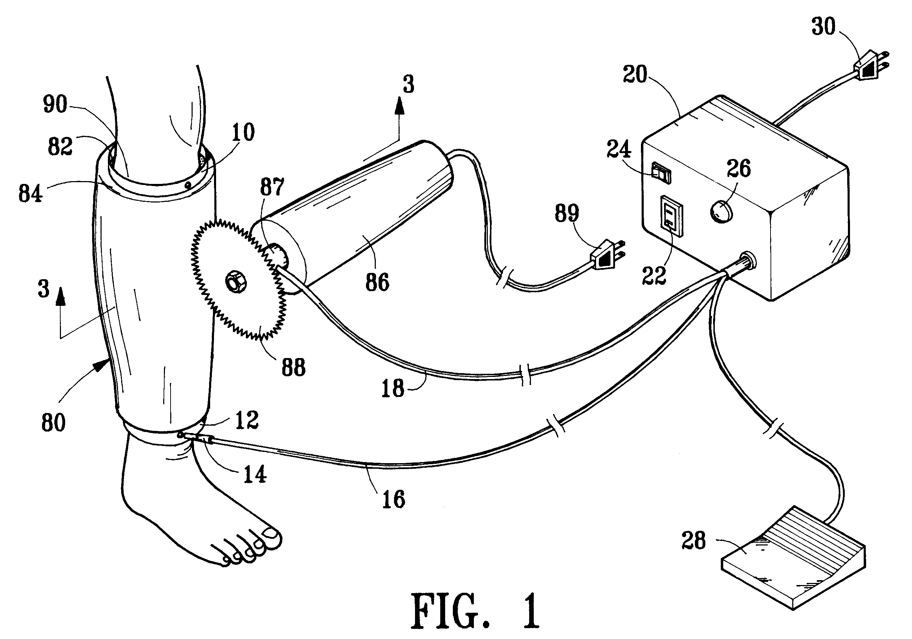

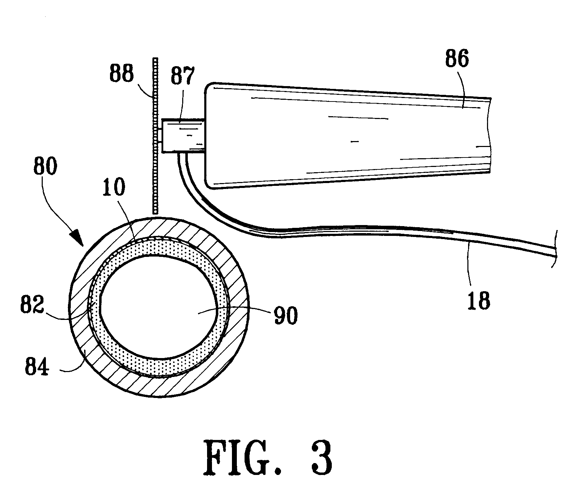

Referring first to Figs. 1 and 3, a plaster cast 80 which is wrapped around a patient's fractured limb 90 has an inner layer 82 composed of tearable absorbent cotton in contact with the patient's skin, an outer layer 84 of a covering material which is hardened upon contact with water, normally plaster or resin for medical purposes, and a thin electrically conductive plate 10 located between the inner layer and the outer layer. References herein to "plaster" are intended also to include other materials such as resins and fibre-glass which can also be used to make casts. The thin conductive plate 10 is made of electrically conductive material such as tin-foil or aluminium foil which does not affect X-ray pictures or diagnosis. The thin conductive plate 10 has an extended end 12 which protrudes outside the plaster cast 80. The extended end 12 is designed to be connected to a control system located in a control box 20 by means of a clamp 14 and a conductive wire 16. An electric saw 86 to cut through the outer layer 84 comprises a high speed motor (not shown in the drawings) and a circular saw blade 88 fixed to a shaft 87 of the motor. The shaft 87 is connected to the control system in the control box 20 by a conductive wire 18 to form a control circuit. The electric saw 86 is connected to a socket 22 on the control box 20 by a plug 89 for electric power to operate the electric saw 86. The control system in the control box 20 is incorporated with a selector switch 24, a warning device 26, and a reset pedal 28. The control box 20 is connected to an external power source by a plug 30. The warning device 26 is an electric or electronic alarm to give an audible or visual warning signal. The warning device is preferably installed on the surface of the control box 20 or the electric saw 86 or other appropriate location so that it can be seen during the sawing process.

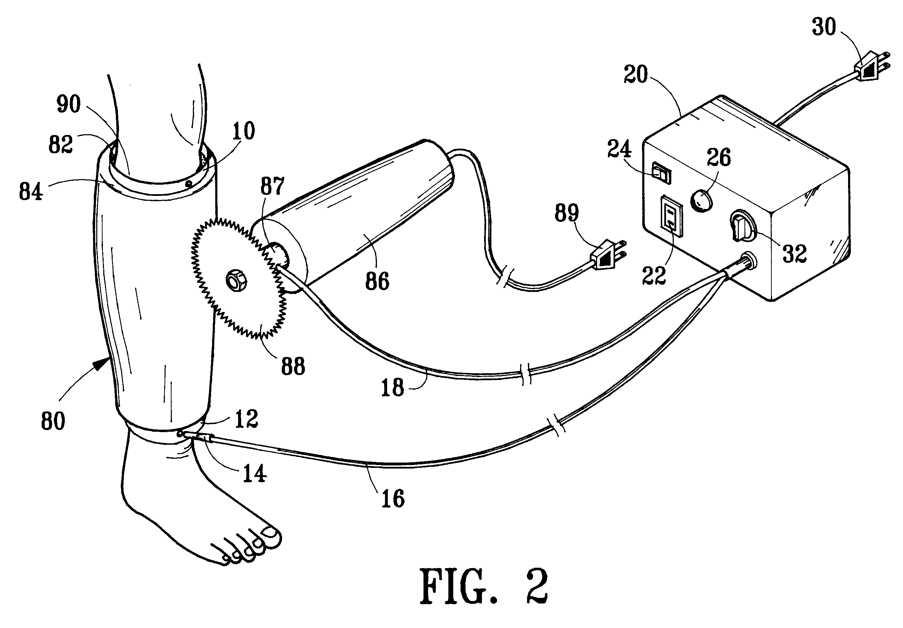

The second embodiment of control system shown in Fig. 2 is the same as the embodiment shown in Fig. 1 except that a reset knob 32 is provided on the control box 20 instead of having a reset pedal 28.

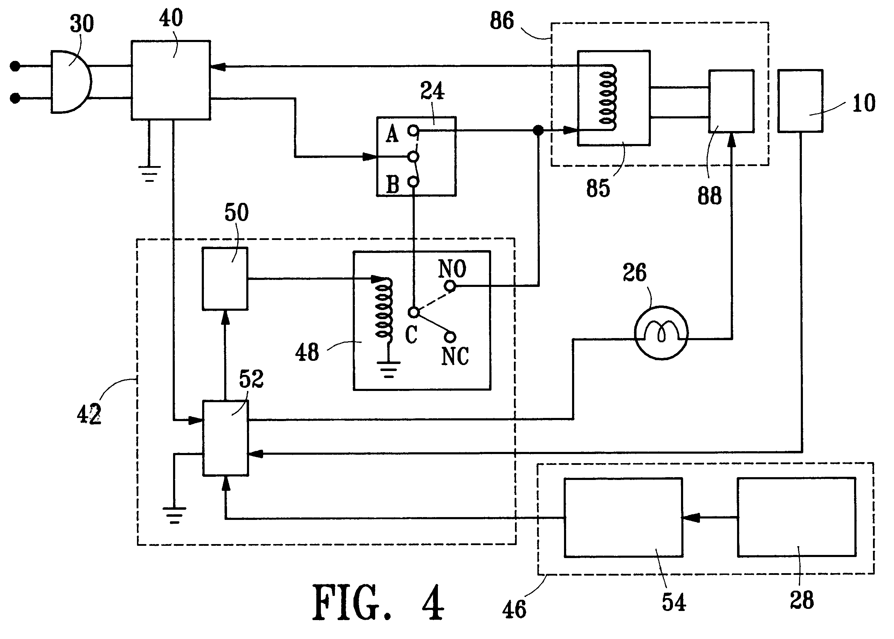

Fig. 4 shows a block diagram for the first embodiment of control system for a plaster cast saw. The control system comprises a power supply 40, a selector switch 24, a relay device 42, a warning device 26, and a reset device 46. The relay device 42 is composed of a relay 48, a driver 50 and a control circuit 52 to cut off the power supply to the electric saw 86 instantaneously as soon as the saw blade 88 makes contact with the thin conductive plate 10. The reset device 46 is composed of the reset pedal 28 and a reset circuit 54 to restart the electric saw 86. The power supply 40 is connected to an external power source for provision of electric power to the electric saw 86 directly, or to the electric saw 86 through the relay device 42, at the choice of the operator. If the selector switch 24 is set to the first contact A, the electric saw is started directly for continuous operation; the relay device 42, the warning device 26 and the reset device 46 do not function. If the selector switch 24 is set to the second contact B, the power is not provided to the electric saw directly, but is connected to a common contact C for the relay 48 which is normally closed at NC so that the electric saw 86 cannot be operated. The power source 40 also provides electric power to the control circuit 52 so that the control circuit is ready to receive signals and upon triggering of the reset device 46 by stepping on the reset pedal 28, which causes the reset circuit 54 to trigger the control circuit 52, and start the driver 50 to drive the relay 48 to trip from the normal closed contact NC to a normal open contact NO. Consequently, the power supply 40 provides the electric saw 86 with electric power through the common contact C and the normal open contact NO for operation of the electric saw. By this means the electric saw 86 can be operated to cut through the plaster cast. Whenever the saw blade 88 makes contact with the thin conductive plate 10 after cutting through the outer layer 84, the saw blade 88, the thin conductive plate 10, the warning device 26 and the control circuit 52 become a closed loop, the warning device 26 is activated to give a warning light or sound, the control circuit 52 cuts off the driver 50 instantaneously so that the relay 48 trips from the normal open contact NO to the normal closed contact NC to cut off power to the electric saw 86, and consequently the electric saw 86 is stopped immediately. To restart the electric saw 86, one only has to depress the reset pedal 28 so that, as described above, the reset circuit 54 triggers the control circuit 52 again to cause the driver 50 to drive the relay 48 to trip from the normal closed contact NC to the normal open contact NO, and start the electric saw 86 again.

The relay 48 can be a solid state device or be mechanically controlled. The warning device shown in the Figures is a visual indicator. It can be replaced by a buzzer to provide an audible warning signal.

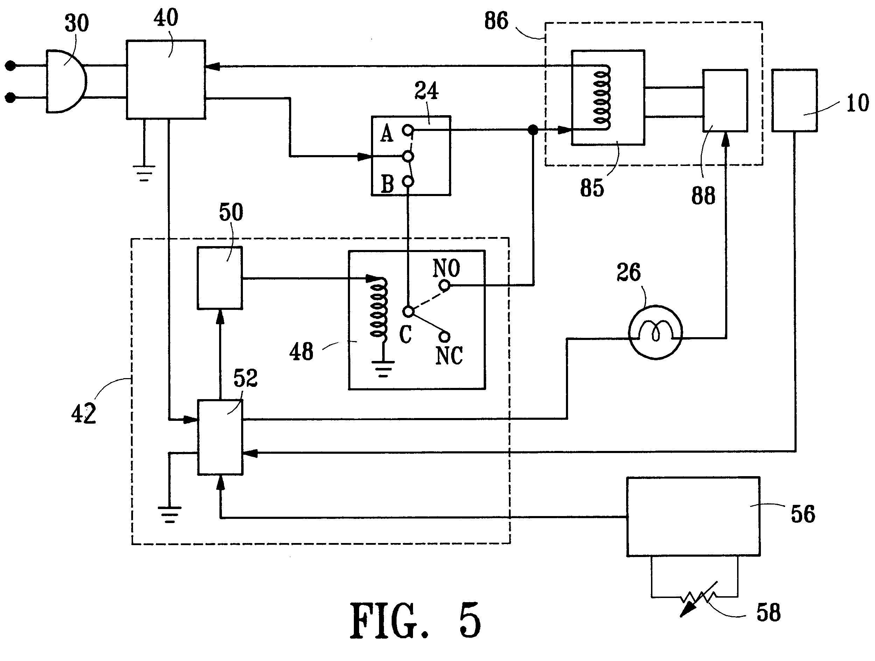

Fig. 5 is a block diagram for the second embodiment of the present invention. The plug 30, the power supply 40, the selector switch 24, the relay control device 42, the warning device 26, the electric saw, and the thin conductive plate 10 are the same as in the first embodiment shown in Fig. 4, but an automatic reset device 56 is used instead of the reset device 46 of Fig. 4. The automatic reset device 56 can be an adjustable pulse generator which is triggered to send a pulse to the relay device 42 on the instant that the saw blade 88 makes contact with the thin conductive plate 10. Upon receipt of the pulse, the relay 48 trips from the normal open contact NO to the normal closed contact NC, and consequently the power supply to the electric saw 86 is cut off. After the passage of a delay time caused by the pulse, the relay 48, in the absence of a pulse input, trips from the normal closed contact NC to the normal open contact NO, and consequently the power supply to the electric saw 86 is restored to continue the cutting process. The length of the delay time can be adjusted by a variable resistor 58. The reset knob 32 on the control box 20 can be turned to adjust the resistance of the variable resistor, and consequently adjust the length of the delay time.

The method, i.e. the process steps, to operate the first embodiment of the electric plaster cast saw control system according to the present invention will now be described:
(1) Connect the control box to an external power source with the plug 30.
(2) Connect the electric saw 86 to the control box 20 by inserting the plug 89 into the socket 22.
(3) Attach the conductive wire 16 to the protruding end 12 of the thin conductive plate with the clamp 14, and attach the other end of the conductive wire 16 to the electric saw 86.
(4) Set the selector switch 24 to contact B position.
(5) Depress the reset pedal 28 to start the electric saw 86. The indicator of the warning device 26 is "off" at this instant, which means that the control box 20 is in good working condition.
(6) Cut through the outer layer 84 of the plaster cast 84 with the electric saw 86. The power source is cut off and the electric saw is stopped immediately, and the indicator of the warning device 26 comes "on" to warn the operator not to operate the electric saw 86, on the instant that the saw blade 88 makes contact with the thin conductive plate 10 so that the patient's skin or flesh is not hurt by the saw blade 88.
(7) If required, depress the reset pedal 28 to resume operation of the electric saw 86. The indicator of the warning device 26 is "off" when the reset pedal 28 is depressed.
(8) Repeat steps (6) and (7) until the plaster cast is completely cut off.
(9) If one wishes to operate the electric saw 86 without using the control system according to the present invention, set the selector switch 24 at the contact A position. Then the electric saw 86 operates continuously and will not stop even when the saw blade 88 makes contact with the thin conductive plate 10.
(10) It is desirable to explain and demonstrate to the patient the whole operating process before sawing is started.

The method of operating the second embodiment of the electric plaster cast saw control system according to the present invention is now described:
(1) to (4) are the same as for the first embodiment.
(5) Turn the reset knob to select the desired length of delay time for interruption of the electric saw 86.
(6) Cut through the outer layer 84 of the plaster cast 80 with the electric saw 86. The power source is cut off and the electric saw 86 is stopped immediately, and the indicator of the warning device 26 comes "on" to warn the operator not to operate the electric saw 86, on the instant that the saw blade 88 makes contact with the thin conductive plate 10 so that the patient's skin or flesh is not hurt by the saw blade 88.
(7) After the passage of the delay time, the power supply to the electric saw 86 is resumed, the electric saw 86 is started again, and the indicator of the warning device 26 is "off".
(8) Repeat steps (6) and (7) until the plaster cast is completely cut off.
(9) If operation of the electric saw 86 without control by the control system according to the present invention is wanted, set the selector switch 24 at the contact A position. Then the electric saw 86 will operate continuously and will not stop even when the saw blade 88 makes contact with the thin conductive plate 10.
(10) It is desirable to explain and demonstrate to the patient the whole operating process before sawing is started.

## Claims

1. An electric plaster cast saw control system for cutting a cast (80) comprising an inner soft layer (82) and an outer layer (84) of hardened material, characterised by
- a thin conductive plate (10) arranged to be located between the inner layer and the outer layer;
- a relay device to cut off power supply to the electric saw on the instant that the thin conductive plate (10) is contacted by the saw blade (88) of the electric saw; and
- a reset device to drive the relay device to resume power supply to the electric saw after the power supply has been cut off.

2. A saw control system as claimed in claim 1, characterised by a warning device (26) to give a visual warning signal on the instant that the saw blade (88) makes contact with the thin conductive plate (10).

3. A saw control system as claimed in claim 1, characterised by a warning device (26) to give an audible signal at the instant that the saw blade (88) makes contact with the thin conductive plate (10).

4. A saw control system as claimed in claim 1, 2 or 3 characterised by a selector switch for selection of a direct connection of the power source to the electric saw so that the electric saw is freed from the control of the relay device.

5. A saw control system as claimed in any preceding claim, characterised in that the reset device comprises a reset circuit to trigger the relay device to resume power supply to the electric saw after the power supply is cut off, and a pedal to activate the reset circuit.

6. A saw control system as claimed in claim 5, characterised in that the reset device is a pulse generator which is triggered to send a pulse to the relay device on the instant that the saw blade makes contact with the thin conductive plate to stop power supply to the electric saw for a certain delay time, and to resume power supply to the electric saw after the end of the delay time.

7. A saw control system as claimed in claim 6, characterised in that the delay time of the pulse generated by the pulse generator can be adjusted by variable resistance means.
